# EUROPEAN PATENT APPLICATION

(11) **EP 2 592 145 A1**
(43) Date of publication of application: **15.05.2013**
(21) Application number: 11188914.3
(22) Date of filing: 11.11.2011
(51) Int. Cl.: C12N 15/113, A61K 31/00, A61P 9/00

(54) **Medicament for the treatment of cardiac disease**

(71) Applicant: Medizinische Hochschule Hannover, 30625 Hannover (DE)
(72) Inventor: Thum, Thomas, 30659 Hannover (DE); Bang, Claudia, 30161 Hannover (DE)
(74) Representative: Taruttis, Stefan Georg

(57) **Abstract**

The present invention relates to a medicament for the treatment of cardiac disease, especially to a medicament suitable for the treatment of myocardial disease involving myocyte hypertrophy, especially fibroblast-derived cardiac hypertrophy, e.g. heart failure, heart hypertrophy, diastolic and/or systolic ventricular dysfunction and/or other cardiovascular diseases, where fibrosis is involved, especially aortic stenosis, atrial fibrillation, genetic forms of cardiomyopathy, cardiac storage diseases by providing an inhibitor specific for at least one of a set of microRNAs found in exosomes originating from cardiac fibroblasts for use as a medicament for human patients

## Description

The present invention relates to a medicament for the treatment of cardiac disease, especially to a medicament suitable for the treatment of myocardial disease involving myocyte hypertrophy, especially fibroblast-derived cardiac hypertrophy, e.g. heart failure, heart hypertrophy, diastolic and/or systolic ventricular dysfunction and/or other cardiovascular diseases, where fibrosis is involved, especially aortic stenosis, atrial fibrillation, genetic forms of cardiomyopathy, cardiac storage diseases (e.g. fabry disease).

### State of the art

Krutzfeldt et al, Nature 685 - 689 (2005) describe the specific inactivation of the microRNA miR-122 using intravenous administration of small interfering double-stranded RNA molecules (siRNA) hybridizing to miR-122. These siRNA molecules complementary to miR-122 could be derivatized by linkage to a cholesterol moiety.

Thum et al, Nature 980 - 984 (2008) describe that miR-21 contributes to myocardial disease by stimulating MAP kinase signalling in fibroblasts. The miRNA inhibitor specific for miR-21 reduced cardiac ERK kinase activity, inhibited cardiac fibrosis and attenuated cardiac dysfunction, validating miR-21 as a disease target in heart failure.

Validi et al, Nature Cell Biology 654 - 659 (2007) describe that mRNA and microRNA molecules are transported from one mast cell to a recipient mast cell in exosomes, involving specificity of the exosome for the recipient cell. In the exosome, miR-1, miR-17, miR-18, miR-181 and miR-375 have been identified and suggested to influence angiogenesis, hematopoiesis, endocytosis, and tumour development.

### Objects of the invention

It is an object of the present invention to provide a compound suitable for use as a medicament in the treatment of cardiac disease, and to provide a method for analyzing cardiac disease.

### General description of the invention

The invention achieves the objects by the features of the claims, especially by providing an inhibitor specific for at least one of a set of microRNAs found in exosomes originating from cardiac fibroblasts, the set of microRNAs comprising or consisting of the 22 microRNAs given in table 1 below, esp. for use as a medicament for human patients. Further, the invention provides a pharmaceutical composition for the prevention or treatment of the cardiac disease, the composition comprising e.g. excipients and formulation agents suitable for medical use, preferably the composition is a liquid, e.g. formulated for intravenous or intraarterial injection. Further, the invention relates to the use of the inhibitors for use in the manufacture of such a pharmaceutical composition. A process for manufacture can e.g. comprise the steps of providing the inhibitor and formulating the composition by incorporating the inhibitor into excipients and/or formulation agents.

The inhibitor preferably is an inhibitor of at least one microRNA from the set of 22 microRNAs given below, especially an inhibitor of miR-21* and/or is an inhibitor of exosomal transport, wherein the exosome in the case of cardiac disease contains at least one microRNA from this set of 22 microRNAs, especially miR-21*. The at least one inhibitor is for use as a medicament in the prevention of treatment of a cardiac disease, especially of cardiac hypertrophy, especially of fibroblast derived cardiac hypertrophy, and/or of cardiac failure, heart hypertrophy, diastolic and/or systolic ventricular dysfunction and/or other cardiovascular diseases, in which fibrosis is involved, especially aortic stenosis, atrial fibrillation, genetic forms of cardiomyopathy, cardiac storage diseases (e.g. fabry disease).

The invention is based on the finding that at least one of this set of microRNAs, especially miR-21*, contributes to this disease when present in cardiomyocytes, whereas the inhibition, elimination or reduction of at least one of these microRNAs in cardiomyocytes by use of the inhibitor according to the invention reduces the disease. A medicament containing the inhibitor of the invention, e.g. when administered to a patient, especially reduces the hypertrophy of cardiomyocytes. This activity of * (star)-microRNA molecules, especially of miR-21 *, is surprising, because it is hitherto generally accepted that the microRNA molecules which are complementary to those microRNAs having an activity (nomenclature without a star) are normally degraded without having an activity; these complementary molecules are generally designated as a * (star)-microRNA.

Based on the finding that the microRNAs comprised in the set of 22 microRNAs as given herein are indicative of cardiac disease, the invention also relates to an analytical process for determining the onset or presence of the cardiac disease, especially of cardiac hypertrophy, by determining the concentration of at least one of the molecules of the set of microRNAs, especially of one of SEQ ID NO: 1 to SEQ NO: 22, preferably of SEQ ID NO: 1.

An inhibitor of at least one of this set of microRNAs, especially of miR-21*, can comprise or consist of an oligonucleotide having specificity for one microRNA of this set of microRNAs, especially for miR-21*, e.g. having a reverse complementary nucleotide sequence hybridizing to the microRNA under physiological or pathophysiological conditions. As it has been found that these microRNAs, especially miR-21*, originate from cardiac fibroblasts and are transported to cardiomyocytes by exosomes originating from cardiac fibroblasts, an inhibitor against one of these microRNAs for the purposes of the invention also comprises an inhibitor of exosome generation, of exosome secretion and/or of exosome transport to cardiomyocytes, especially an inhibitor specific for exosome generation, exosome excretion by cardiac fibroblasts, e.g. an agent reducing or inhibiting the generation and/or excretion of exosomes containing at least one microRNA of this set of microRNAs by cardiac fibroblasts, and/or an agent reducing or eliminating the transport of exosomes comprising at least one of these microRNAs to cardiomyocytes.

Inhibitors of microRNAs preferably have a sequence hybridizing to the microRNA, e.g. a sequence having at least 90%, preferably 95%, more preferably 99% identity to a reverse complementary nucleotide sequence of the microRNA, which inhibitory nucleotide sequence is e.g. comprised in a short hairpin RNA (shRNA) or in a pre-microRNA.

**Table 1: set of microRNAs and preferred specific inhibitory oligonucleotide sequences**

| microRNA | SEQ ID NO: sequence | Inhibitory oligonucleotide SEQ ID NO and sequence |
|---|---|---|
| hsa-miR-21 * | SEQ ID NO: 1 | SEQ ID NO: 23 |
| | CAACACCAGUCGAUGGGCU GU | ACAGCCCAUCGACUGGUGUUG |
| hsa-let-7a | SEQ ID NO: 2 | SEQ ID NO: 24 |
| | UGAGGUAGUAGGUUGUAUA GUU | AACUAUACAACCUACUACCUC A |
| hsa-miR-9* | SEQ ID NO: 3 | SEQ ID NO: 25 |
| | AUAAAGCUAGAUAACCGAA AGU | ACUUUCGGUUAUCUAGCUUUA U |
| hsa-miR-17-5p | SEQ ID NO: 4 | SEQ ID NO: 26 |
| | CAAAGUGCUUACAGUGCAG GUAG | CUACCUGCACUGUAAGCACUU UG |
| hsa-miR-21 | SEQ ID NO: 5 | SEQ ID NO: 27 |
| | UAGCUUAUCAGACUGAUGU UGA | UCAACAUCAGUCUGAUAAGCU A |
| hsa-miR-23a | SEQ ID NO: 6 | SEQ ID NO: 28 |
| | AUCACAUUGCCAGGGAUUU CC | GGAAAUCCCUGGCAAUGUGAU |
| hsa-miR-30b-3p | SEQ ID NO: 7 | SEQ ID NO: 29 |
| | CUGGGAGGUGGAUGUUUAC UUC | GAAGUAAACAUCCACCUCCCA G |
| hsa-miR-129* | SEQ ID NO: 8 | SEQ NO: 30 |
| | AAGCCCUUACCCCAAAAAG CAU | AUGCUUUUUGGGGUAAGGGC UU |
| hsa-miR-132 | SEQ ID NO: 9 | SEQ ID NO: 31 |
| | UAACAGUCUACAGCCAUGG UCG | CGACCAUGGCUGUAGACUGUU A |
| hsa-miR-135a | SEQ ID NO: 10 | SEQ ID NO: 32 |
| | UAUGGCUUUUAUUCCUAU GUGA | UCACAUAGGAAUAAAAAGCCA UA |
| hsa-miR-135b | SEQ ID NO: 11 | SEQ ID NO: 33 |
| | UAUGGCUUUCAUUCCUAU GUGA | UCACAUAGGAAUGAAAAGCCA UA |
| hsa-miR-138 | SEQ ID NO: 12 | SEQ ID NO: 34 |
| | AGCUGGUGUUGUGAAUCAG GCCG | CGGCCUGAUUCACAACACCAG CU |
| hsa-miR-181a | SEQ ID NO: 13 | SEQ ID NO: 3 5 |
| | AACAUUCAACGCUGUCGGU GAGU | ACUCACCGACAGCGUUGAAUG UU |
| hsa-miR-181b | SEQ ID NO: 14 | SEQ ID NO: 36 |
| | AACAUUCAUUGCUGUCGGU GGGU | ACCCACCGACAGCAAUGAAUG UU |
| hsa-miR-181c | SEQ ID NO: 15 | SEQ ID NO: 37 |
| | AACAUUCAACCUGUCGGUG AGU | ACUCACCGACAGGUUGAAUGU U |
| hsa-miR-181d | SEQ ID NO: 16 | SEQ ID NO: 3 8 |
| | AACAUUCAUUGUUGUCGGU GGGU | ACCCACCGACAACAAUGAUGU U |
| rno/mmu-miR- | SEQ ID NO: 17 | SEQ ID NO: 39 |
| 341 | UCGGUCGAUCGGUCGGUCG GU | ACCGACCGACCGAUCGACCGA |
| hsa-miR-374 | SEQ ID NO: 18 | SEQ ID NO: 40 |
| | AUAUAAUACAACCUGCUAA GUG | CACUUAGCAGGUUGUAUUAUA U |
| hsa-miR-375 | SEQ ID NO: | SEQ ID NO: 41 |
| | UUUGUUCGUUCGGCUCGCG UGA | UCACGCGAGCCGAACGAACAA A |
| hsa-miR-539 | SEQ ID NO: 20 | SEQ ID NO: 42 |
| | GGAGAAAUUAUCCUUGGUG UGU | ACACACCAAGGAUAAUUUCUC C |
| hsa-miR-758 | SEQ ID NO: 21 | SEQ ID NO: 43 |
| | UUUGUGACCUGGUCCACUA ACC | GGUUAGUGGACCAGGUCACAA A |
| hsa-miR-592 | SEQ ID NO: 22 | SEQ ID NO: 44 |
| | UUGUGUCAAUAUGCGAUGA UGU | ACAUCAUCGCAUAUUGACACA A |

Sequences are given from 5'to 3'. The inhibitory sequences are specific for the microRNA given in the same row of the left column.

Preferably, the nucleotide sequences inhibitory to the set of microRNAs are DNA, RNA, peptide linkage oligonucleotides (PNA), preferably single-stranded. For producing inhibitors to one of the microRNAs of the set of microRNAs, the inhibitory sequence can be produced by de novo synthesis, e.g. on a solid support followed by hydrolysing the bond to the solid support, or by copying a nucleotide template, e.g. using PCR, or by transcription from a DNA template encoding the inhibitory compound, e.g. a nucleotide sequence corresponding to one of the set of microRNAs.

The inhibitory oligonucleotide preferably is comprised in or consists of a single-stranded nucleotide sequence in order to exclude undesired effects of the nucleotide sequence that is complementary to the inhibitory oligonucleotide.

Optionally, the inhibitory oligonucleotide specific for one of the set of microRNAs can be provided with a chemical modification resulting e.g. in a stabilization such as 2-O-methyl-modification of the oligonucleotide, LNA-modification wherein the 2-O-oxygen is connected to the 4-position by a methylene linker, resulting in a tight bicycle and locking into a C3-endo (RNA) sugar conformation, preferably as described by Elmen et al (LNA-mediated microRNA silencing in non-human primates. Nature 452: 896-899 (2008a)), Krutzfeldt et al, (Silencing of microRNAs in vivo with 'antagomirs'. Nature 438: 685-689 (2005)) and/or by Krutzfeldt et al, (Specificity, duplex degradation and subcellular localization of antagomirs. Nucleic Acids Res 35: 2885-2892 (2007)). In these modified oligonucleotides it is preferred that additionally the balance between phosphodiester- and phosphorothioate linkages between the nucleotides is obtained for the oligonucleotide stability as phosphorothioates show improved stability as described e.g. by (Faria & Ulrich, Curr Opin Mol Ther 10: 168-175 (2008)). The 2-O-methyl modification is preferred as it results in high oligonucleotide stability and efficacy. As another chemical modification, a cholesterol moiety can be linked to the oligonucleotide to improve cellular uptake as described by (Krutzfeldt et al, 2005), and by Thum et al (Nature 456: 980-984(2008b)).

In a second embodiment, the inhibitor of one of the microRNAs of the set of 22 microRNAs, especially against miR-21*, is an inhibitor of exosome generation, of exosome excretion, or of exosome transport to cardiomyocytes. Preferred inhibitors of exosome generation and/or of exosome transport to cardiomyocytes are specific for exosomes originating from fibroblasts. Preferred inhibitors of exosome formation and/or exosome secretion are selected from the group comprising or consisting of the neutral sphingomyelinase 2-inhibitor GW4869, brefeldin, dimethylamiloride (DMA), manumycin A, spiroepoxide, gluthathione, and combinations thereof.

### Detailed description of the invention

The invention is now described in greater detail by way of examples with reference to the figures, wherein
- Figure 1 shows an electron-microscopic picture of exosomes isolated by ultracentrifugation from fibroblast culture supernatant,
- Figure 2 shows micrographs of cardiomyocytes contacted with labelled exosomes isolated from rat cardiac fibroblast culture in confocal microscopy, in A) following cultivation for 30 min, and in B) following cultivation for 2 h,
- Figure 3 shows the analysis of cardiomyocytes after overexpressing miR-21 * for 72h, in A) relative levels of expression of miR-21* (mir-21*), in B) measurements of cardiomyocyte size, and in C) light micrographs of cardiomyocytes after over-expression of pre - miR-21* and of a comparative non-specific microRNA (neg pre - miR).
- Figure 3D shows cell sizes of cardiomyocytes after incubation with exosomes generated by rat cardiac fibroblasts overexpressing pre - mir-21* or overexpressing a comparative non-specific microRNA (neg pre-miR),
- Figure 3E shows light micrographs of cardiomyocytes after incubation with exosomes generated by rat cardiac fibroblasts overexpressing pre - mir-21* or overexpressing a comparative non-specific microRNA (neg pre-miR),
- Figure 4 shows the increase of excretion of exosomes containing microRNAs as indicated from cardiac fibroblasts upon induction of hypertrophy,
- Figure 5 shows the analysis of cardiomyocytes after incubation with exosomes generated by rat cardiac fibroblasts transfected with anti - miR-21* in comparison cardiomyocytes incubated with exosomes generated by fibroblasts transfected with a non-specific anti - microRNA (neg anti - miR), in A) the cardiomyocyte area, and in B) micrographs,
- Figure 6 shows the analysis of the effect of inhibiting exosome excretion from cardiac fibroblasts after 48 hours, in A) a micrograph of rat cardiac fibroblasts treated with control compound, and B) a micrograph of rat cardiac fibroblasts cultivated in the presence of an nSmase2 inhibitor,
- Figure 7 shows micrographs of rat cardiac fibroblasts treated with nSmase2 inhibitor in comparison to a control, in phalloidin - actin staining, in staining for CD63 - positive microvesicles and in an overlay, in A), B) and C) for control - treated fibroblasts, and in D), E) and F) in fibroblasts treated with nSmase2 inhibitor, respectively, and
- Figure 8 shows graphical presentations of the reduction of representative microRNA secreted from cardiac fibroblasts after treatment with nSmase2 inhibitor for 48 h.

Cardiac cells were represented by cardiac fibroblasts and cardiomyocytes isolated from newborn rats. Primary cardiac fibroblasts were cultured in Dulbecco's modified Eagle's medium (DMEM), supplemented with 10% fetal calf serum (FCS) and 1% penicillin / streptomycin at 37 °C in a 5% CO₂ atmosphere under cell culture conditions. Primary cardiomyocytes were cultivated in minimal essential medium (MEM) containing vitamin B12, NaHCO₃ and 5% FCS at 37°C in a 1% CO₂ atmosphere under cell culture conditions. For exosome isolation from supernatant, the medium was changed to DMEM supplemented with 0.1% exosome - depleted FCS. Supernatant from fibroblasts was collected for 3 days, and exosomes were isolated by differential centrifugation, comprising centrifugation at 300 x g for 10 min, 2000 x g for 10 min and 10,000 x g for 30 min, followed by filtration through a 0.22 µm filter for eliminating cells, dead cells and cellular debris. The supernatant was ultracentrifuged for exosome purification at 100,000 x g for 90 min followed by an additional washing step of the exosome pellet in phosphate buffered saline (PBS) at 100,000 x g for 90 min. The exosome pellet was resuspended in 100 - 130 µL PBS and stored at -80°C. In the alternative, exosomes were isolated using the Exoquick exosome precipitation solution (obtainable from SBI). The protein content of exosomes was measured using the micro-BCA protein assay (obtainable from ThermoScientific).

It was found that exosomes isolated from a fibroblast culture supernatant (n=2) using ultracentrifugation had following protein content:

| | |
|---|---|
| protein/cell (µg/10⁶ cells) | 1.52 (SD=0.52) |
| protein/vol. cell suspension (µg/µL) | 0.81(SD=0.23) |

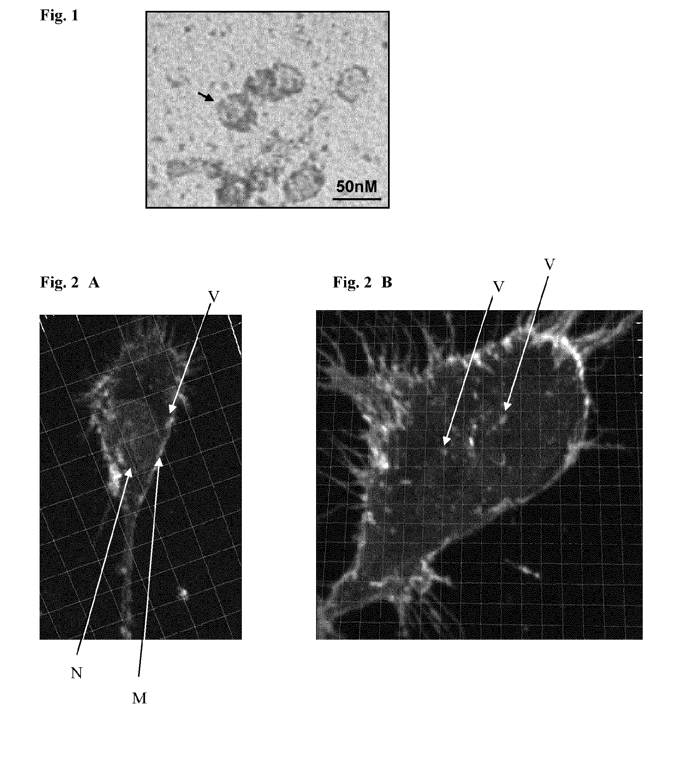

Figure 1 shows an electron microscopic picture of cardiac fibroblast - derived exosomes (arrow), having a diameter in the range of 30 - 50 nm.

For electron microscopy, exosomes (e.g. 10 µg) were loaded onto carbon - coated grids (Formvar), fixed in 2% paraformaldehyde and washed, followed by 1% glutaraldehyde, washing and contrasting in uranyl oxalate solution, then embedded in 4% uranyl acetate and 2% methylcellulose. Examination was in an electron microscope (Carl Zeiss NTS).

Exosomes isolated from cardiac fibroblasts were labelled with the lipophilic dye PKH26. Labelled fibroblast - derived exosomes were added to cultivated cardiomyocytes and incubated for 30 min or 2 hours. Figure 2A in a confocal micrograph shows labelled exosomes (V) that were taken up by cardiomyocytes after 30 min (grid approx. 4µm). Figure 2B (grid approx. 2µm) in a confocal micrograph shows that 2 h after incubation of the cardiomyocytes with the labelled fibroblast - derived exosomes an increased number of exosomes is taken up by cardiomyocytes, with fusion or cluster formation between exosomes within cardiomyocytes. Membrane staining was by agglutinin-Alexa488 (green, M) the nucleus was stained with DAPI (blue, N).

Labelling of exosomes was done using the PKH26 red fluorescence linker kit (obtainable from Sigma) according to the manufacturer's protocol with minor modifications. Briefly, exosomes diluted in PBS were added to 1 mL diluent C. 4 µL of the PKH26 dye was added to 1 mL diluent C and mixed with the exosome dilution, followed by adding 2 mL 0.5% BSA to allow binding of excess dye. Samples were washed at 100,000 x g for 1 h. The pellet was suspended in 100 µL PBS. For confocal microscopy, cardiomyocytes were grown on glass cover slips by cultivation in cardiomyocyte medium. 7.5 µg labelled exosomes were added to 100,000 cardiomyocytes and incubated at 37°C for 30 min or 2 h. Cells were then washed twice with PBS, fixed in 4% paraformaldehyde for 15 min, washed twice with PBS and stained with agglutinin-Alexa488 for 30 min for labelling of the cell membrane, washed twice with PBS, and embedded with Vector shield mounting medium with DAPI for nuclear staining.

The effect of the set of microRNAs on the size of cardiomyocytes is shown on the example of miR-21* by over-expression miR-21* in cardiomyocytes. In short, cardiomyocytes were grown in 6-well plates to 80% confluence and were transfected using lipofectamine with 100 nM pre - miR-21* oligo-nucleotide or with a non-specific precursor microRNA (negative precursor used as control). 4 h after transfection, the medium was discarded and replaced by fresh medium. After 48 hours RNA was isolated by TRIfast reagent, analysis of RNA was performed by quantitative real-time polymerase chain reaction (qRT-PCR) using TaqMan primers specific for miR-21*. The TaqMan miRNA assay from AppliedBiosystems was used according to the manufacturer's protocol, using a total of 15 ng total RNA for reverse transcription of each microRNA (miR-21* and the internal control RNAs). For qRT-PCR analysis, samples were pre-diluted 1:3 with nuclease-free water, generating an mRNA standard curve at 1:5, 1:25, 1:125 using 2 µL cDNA template for qRT-PCR. For cell size measurement, cells were fixed in 4% paraformaldehyde and stained with alpha-actinin and DAPI.

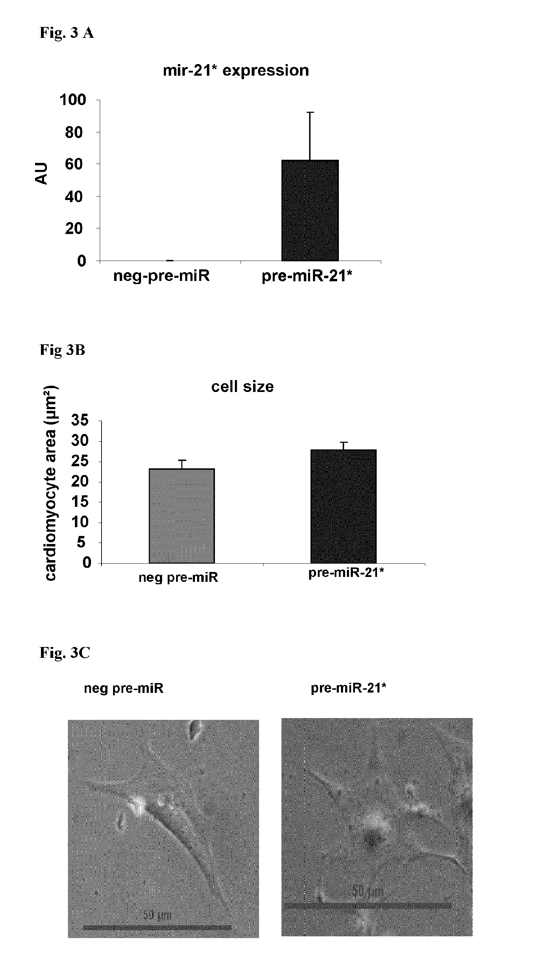

The effect of the transfection with the miR-21* in comparison to the control (neg pre miR) on the expression of miR-21* is shown in Figure 3A. As expected, the transfection with pre - miR-21* gave an increase of miR-21* concentration (miR-21* expression) in comparison to the control (neg-pre-miR).

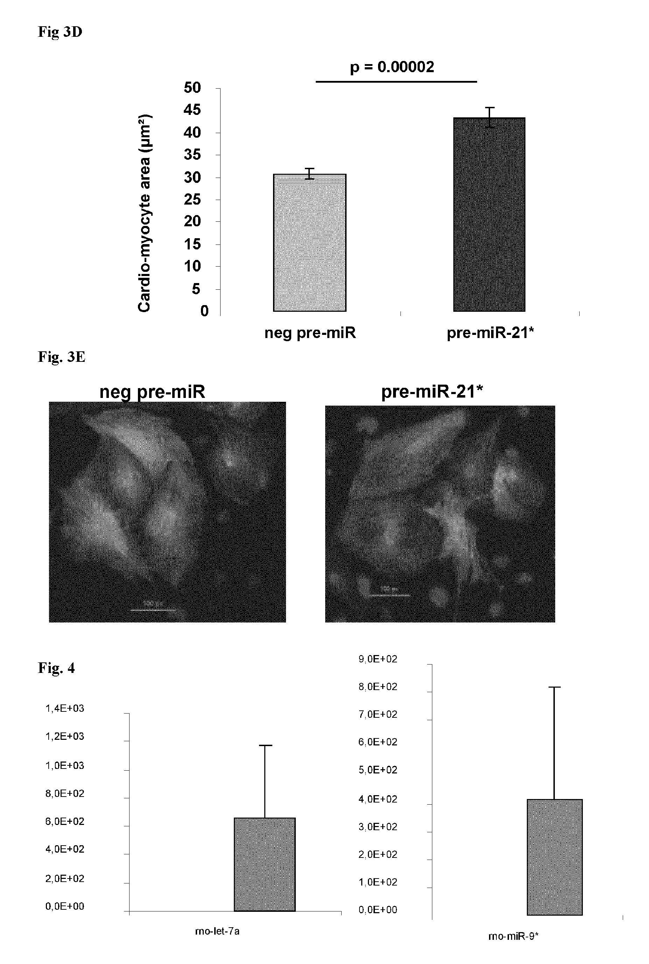

Figure 3B shows the cell size of cardiomyocytes for the control transfected and for the pre - miR-21* transfected cardiomyocytes, showing an increase in cells size in response to the increased concentration of miR-21*. The micrographs of Figure 3C confirm the increase in cardiomyocyte size is due to the presence ofmiR-21* over the control transfected cardiomyocytes (neg precursor). In Figure 3D, the cell size of cardiomyocytes in response to exosomes that were isolated from rat cardiac fibroblast-derived overexpressing pre-miR-21* is shown to lead to an increase in cell size. Figure 3E shows micrographs confirming that the microRNA leading to an increase in cardiomyocyte cell size is transferred from cardiac fibroblasts by way of exosomes to the cardiomyocyte.

These analytical data show that miR-21* as an exemplary microRNA of the set of microRNAs is generated in cardiac fibroblasts, which are taken up by cardiomyocytes, resulting in an increase in cell size of cardiomyocytes.

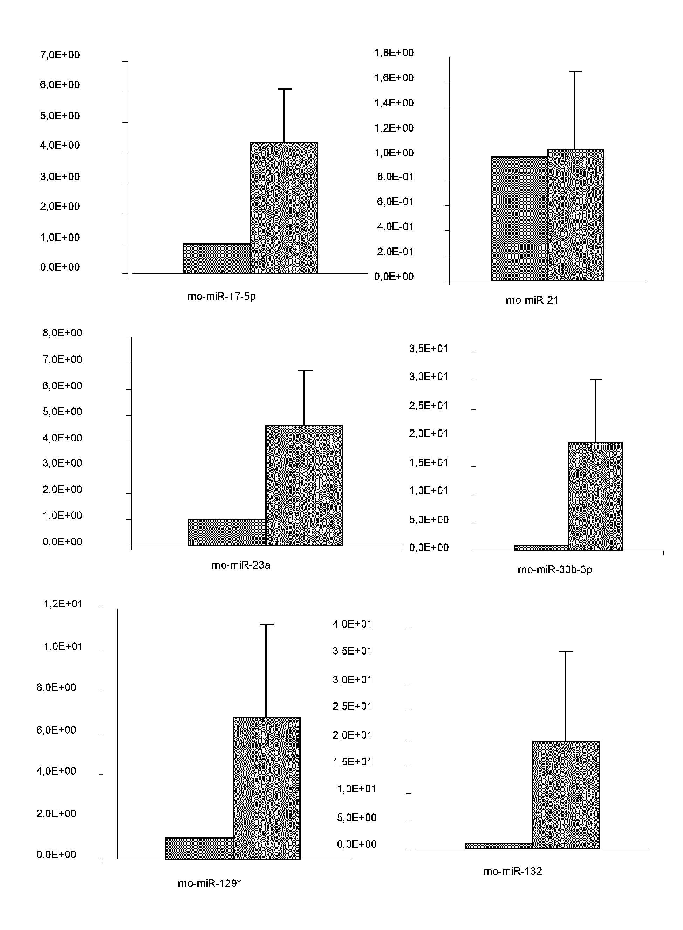

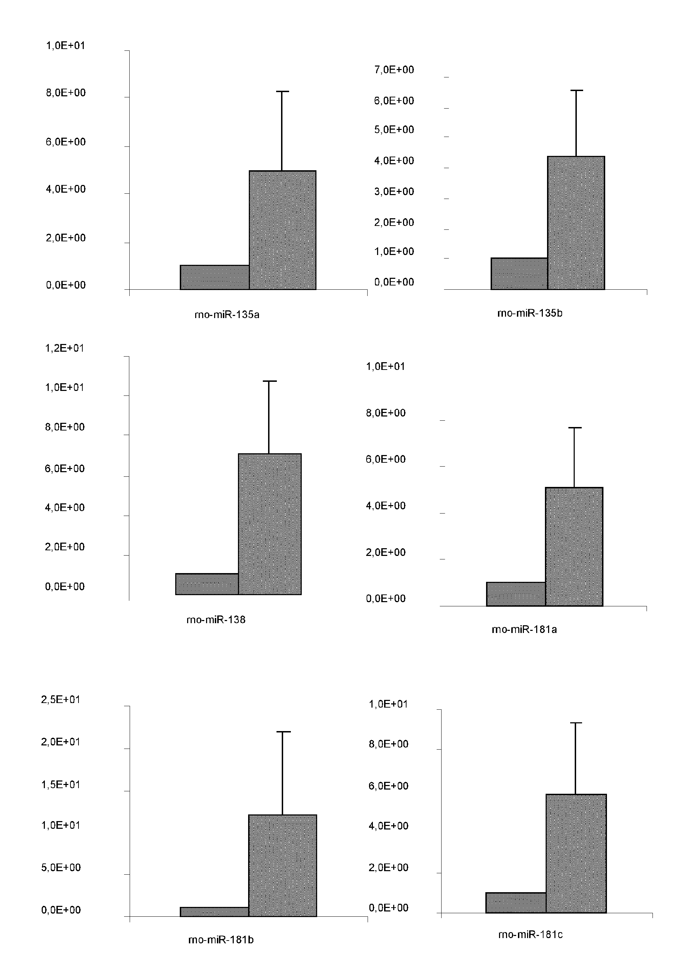

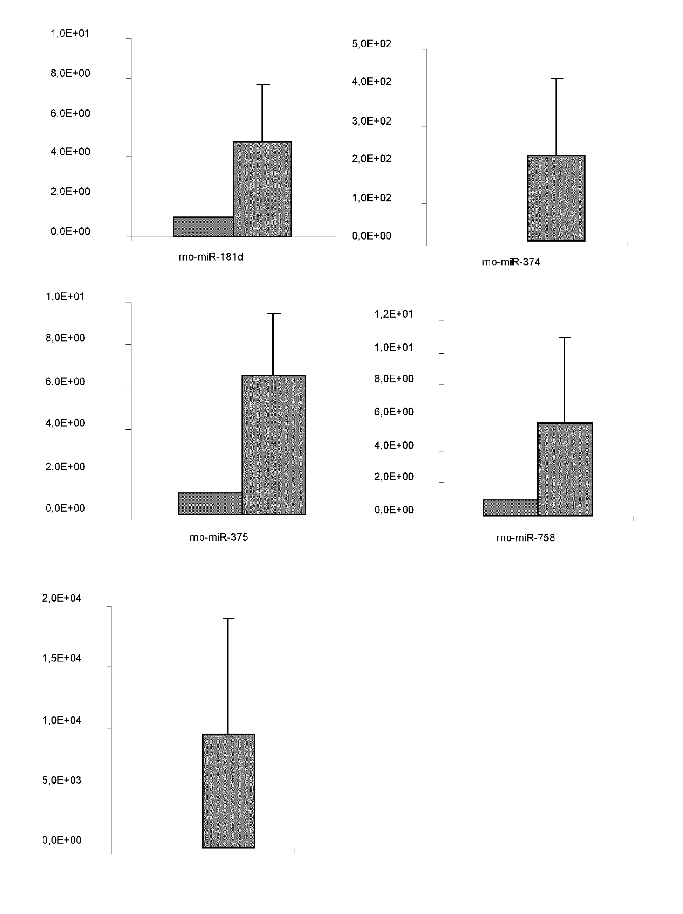

Figure 4 shows graphical representations of concentrations of the microRNAs that were identified in exosomes isolated from the supernatant of cultivated cardiac fibroblasts without induction (left hand columns) and after imparting a hypertrophy stimulus using angiotensin II plus interleukin-1β. Induction of hypertrophy using 1 nM Angiotensin II and 4 ng/mL IL-1β for 24 h in cardiac fibroblasts stimulated an increase of microRNAs of the set of 22 microRNAs, exemplified by miR-21*, miR-341, and miR-539. Results of qRT-PCR on total RNA of exosomes isolated from the fibroblast culture supernatant are shown in Figure 4, left col. for the non-induced control culture, right column for the culture induced by Angiotensin II plus IL-1β. The increase of these microRNAs in exosomes generated from fibroblasts upon a stimulus of cardiac injury provides a basis that these microRNAs participate in the induction of cardiomyocyte hypertrophy, and hence an inhibitor of one of these microRNAs is suitable as a medicament in the treatment of cardiac diseases involving cardiomyocyte hypertrophy.

### Example 1: Inhibition of miR-21* decreases cardiomyocyte cell size

Cardiomyocytes were incubated with rat cardiac fibroblast-derived exosomes, wherein the fibroblasts were transfected with an oligonucleotide inhibitor specific for miR-21* (anti- miR-21*, having SEQ ID NO: 23), or with a non-specific anti-miR (Applied Biosystems, AM17010) for comparison.

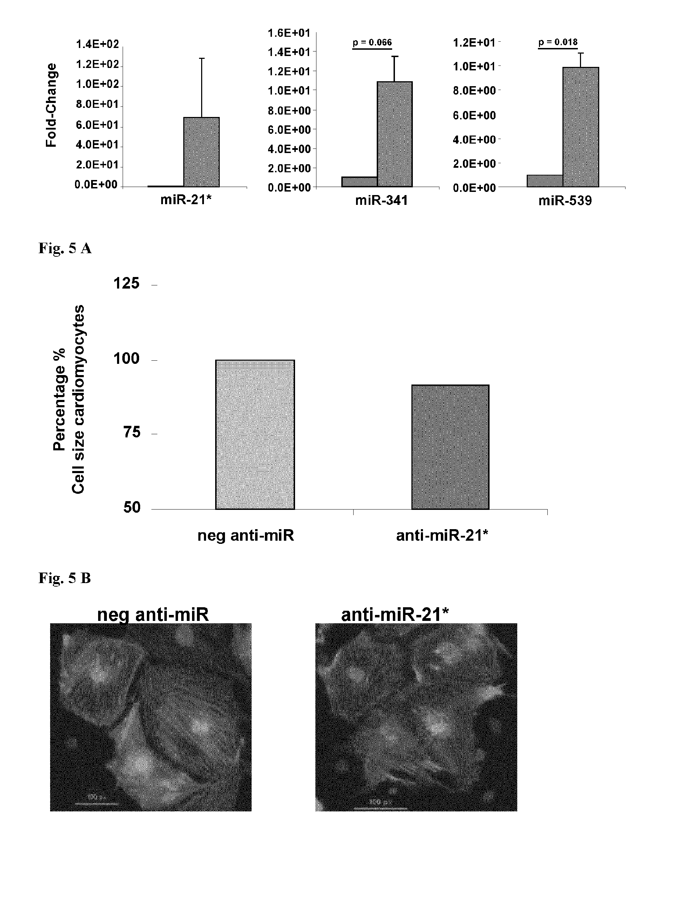

Cardiomyocytes were incubated with exosomes obtained from rat cardiac fibroblasts which had been transfected with anti-miR-21* for 72 h prior to analysis. Figure 5A shows the reduction of the cell size of cardiomyocytes upon presence of an inhibitor of miR-21* in comparison to control-transfected rat cardiac fibroblast-derived exosomes. Figure 5B shows the microscopic analysis of cardiomyocytes incubated with the exosomes isolated from control-transfected rat cardiac fibroblasts (neg anti miR) or with exosomes isolated from fibroblasts isolated from anti-miR-21 *-transfected rat cardiac fibroblast-derived exosomes for 72 hours. Staining of the cytoskeleton was with anti-actinin, and nuclear staining was with DAPI.

On the example of an inhibitor of miR-21*, these data show that an inhibitor directed against one of the microRNAs of the set of 22 microRNAs results in a comparative size reduction of cardiomyocytes, e.g. in a reduction of the cardiomyocyte hypertrophy induced by cardiac fibroblast-derived exosomes. This shows that presence of an inhibitor against at least one microRNA of the set of microRNAs leads to the inhibition of fibroblast-derived microRNAs and is effective against cardiac disease, especially against cardiac hypertrophy.

Further, this example shows that the inhibitor can be provided by providing the inhibitor to cardiac fibroblasts, e.g. by contacting these fibroblasts with the inhibitor, e.g. by transfection, as the inhibitor is transported to the cardiomyocytes by exosomes excreted by the fibroblasts.

### Example 2: Inhibitor of exosome excretion from cardiac fibroblasts is effective against cardiomyocyte hypertrophy

The activity of an inhibitor against generation and/or against excretion of exosomes by cardiac fibroblasts is shown by an exemplary inhibitor of exosome excretion, resulting in the reduction or prevention of induction of cardiomyocyte hypertrophy.

Cardiac fibroblasts have been found to express neutral sphingomyelinase 2 (nSmase2), e.g. using Western blotting on isolated fibroblasts (15 - 20 µg protein) with a primary antibody against nSmase (Santa Cruz, sc-67305) and antibody directed against GAPDH (Abcam, ab8245) as a control, followed by a secondary detection antibody.

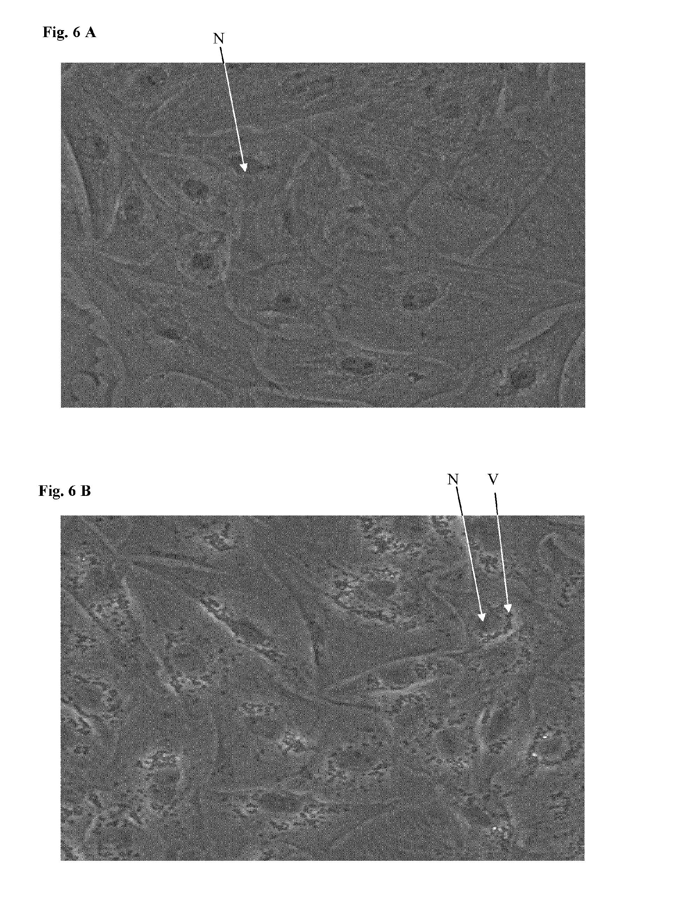

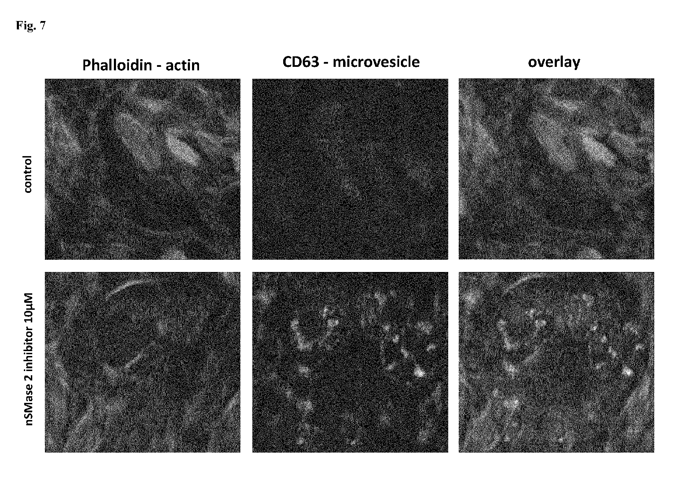

Cultivated cardiac fibroblasts were treated for 48 h with 10 µM GW4869, which is an nSmase2 inhibitor. Analysis of cells using brightfield microscopy is shown in Figure 6A for control without nSmase2 inhibitor, and in Figure 6B for the fibroblasts treated with nSmase2 inhibitor. The bright field image shown in Figure 6B demonstrates the accumulation of vesicles/exosomes (V) within fibroblasts in the presence of the nSmase2 inhibitor (N = nucleus). Immunofluorescent staining against CD 63, which is an exosome - specific marker reveals the accumulation of CD 63 - positive vesicles in cardiac fibroblasts as shown in Figure 7.

From the fibroblast cultures without and with the nSmase2 inhibitor, the microRNAs was analysed in exosomes isolated from the culture supernatant. For the quantitative RT-PCR, the QuantiMirTM RT kit available from SystemBiosciences (SBI) was used. Total RNA was isolated, and all small non-coding RNAs were converted into cDNA after addition of a polyA tail to the 3'-end in the presence of polyA polymerase, using an oligo-dT primer to initiate reverse transcription into cDNA. This cDNA was analysed in quantitative real-time PCR (qRT-PCR) with a 3' universal reverse-primer labelled 2x with SybrGreen, and a specific microRNA assay primer. For control, primers specific for endogenous RNA were included in each assay, namely for rno-U6 (CGCAAAUUCGUGAAGCGUUC SEQ ID NO: 45), for RNU43 (CUUAUUGACGGGCGGACAGAAAC SEQ ID NO: 46) and for U1 (CGACUGCAUAAUUUGUGGUAGUGG SEQ ID NO: 47). A total of 30 - 100 ng RNA was used. CT values used for microRNAs were obtained from excreted exosomes were normalized to the endogenous controls of the cardiac fibroblasts.

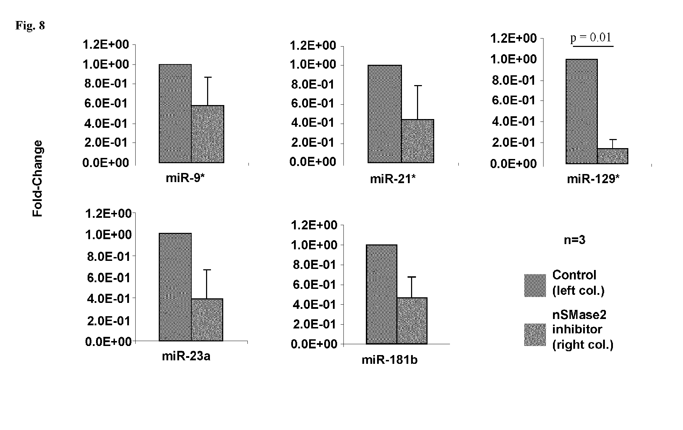

Using quantitative RT-PCR it was found that the inhibition of exosome excretion lead to a significant reduction of secretion of fibroblast derived microRNAs, especially of miR-21*, of miR-129*, of miR-9*, of miR-23a and of miR-181b. These results are graphically shown in Figure 8.

## Claims

1. Compound for use as a medicament in the prevention or treatment of a cardiac disease, **characterized in that** the compound is an inhibitor of a cardiac fibroblast - derived microRNA or an inhibitor of the generation and/or excretion of exosomes from cardiac fibroblasts.

2. Compound according to claim 1, **characterized in that** the inhibitor of a fibroblast - derived microRNA comprises a nucleotide sequence specifically hybridizing to at least one of the microRNAs selected from the set of microRNAs consisting of SEQ ID NO: 1 (miR-21*) to SEQ ID NO: 22.

3. Compound according to claim 2, **characterized in that** the inhibitor is a single-stranded oligonucleotide comprising a nucleotide sequence having a sequence identity of at least 90% to at least one of SEQ ID NO: 23 to SEQ ID NO: 44.

4. Compound according to one of the preceding claims, **characterized in that** the compound is derivatized by at least one moiety selected from cholesterol, by the 2-O-methyl-modification of the oligonucleotide, and/or by the LNA-modification of the oligonucleotide, wherein the 2-O-oxygen is connected to the 4-position by a methylene linker.

5. Compound according to claim 1, **characterized in that** the inhibitor is specific for neutral sphingomyelinase 2 is selected from the group GW4869, brefeldin, dimethylamiloride (DMA), manumycin A, spiroepoxide, gluthathione, and combinations thereof.

6. Compound according to one of the preceding claims, **characterized in that** the compound is formulated as a liposome or as an exosome excreted from a fibroblast provided with the inhibitor.

7. Compound according to one of the preceding claims, **characterized in that** the cardiac disease is a myocardial disease involving myocyte hypertrophy, fibroblast-derived cardiac hypertrophy, heart failure, heart hypertrophy, diastolic and/or systolic ventricular dysfunction and/or a cardiovascular disease involving fibrosis, aortic stenosis, atrial fibrillation, genetic forms of cardiomyopathy, cardiac storage diseases and/or fabry disease.

8. Use of a compound for the production of a medicament for the prevention or treatment of a cardiac disease, **characterized in that** the compound is an inhibitor of a cardiac fibroblasts - derived microRNA or an inhibitor of the generation and/or of the excretion of exosomes from cardiac fibroblasts.

9. Use according to claim 7, **characterized in that** the inhibitor of a fibroblast - derived microRNA comprises a nucleotide sequence specifically hybridizing under cell physiological conditions to at least one of the microRNAs selected from the set of microRNAs consisting of SEQ ID NO: 1 (miR-21*) to SEQ ID NO: 22.

10. Use according to one of claims 8 to 9, **characterized in that** the inhibitor is specific for neutral sphingomyelinase 2.

11. Use according to one of claims 7 to 9, **characterized in that** the cardiac disease is a myocardial disease involving myocyte hypertrophy, fibroblast-derived cardiac hypertrophy, heart failure, heart hypertrophy, diastolic and/or systolic ventricular dysfunction and/or a cardiovascular disease involving fibrosis, aortic stenosis, atrial fibrillation, genetic forms of cardiomyopathy, cardiac storage diseases and/or fabry disease.

12. Process for analysis of the onset or presence of cardiac disease, **characterized by** detection of at least one microRNA from the set of microRNAs consisting of SEQ ID NO: 1 (miR-21*) to SEQ ID NO: 22.

13. Process according to claim 8, **characterized in that** the microRNA is detected in a cell-free sample fraction obtained from a patient.


SEQUENCE LISTING

<110> Medizinische Hochschule Hannover

<120> Medicament for the treatment of cardiac disease

<130> M1047EP

<160> 47

<170> BiSSAP 1.0

<210> 1
<211> 21
<212> RNA
<213> Homo sapiens

<220>
<221> source
<222> 1..21
<223> /mol_type="RNA"
      /organism="Homo sapiens"

<220>
<221> ncRNA
<222> 1..21
<223> /ncRNA_class="siRNA"
      /note="SEQ ID NO:1 = hsa-miR-21*"

<400> 1
caacaccagu cgaugggcug u                                                21


<210> 2
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 2 = hsa-let-7a"

<400> 2
ugagguagua gguuguauag uu                                               22


<210> 3
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 3 = hsa-miR9*"

<400> 3
auaaagcuag auaaccgaaa gu                                                22


<210> 4
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 4 = hsa-miR-17-5p"

<400> 4
caaagugcuu acagugcagg uag                                               23


<210> 5
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 5 = hsa-miR-21"

<400> 5
uagcuuauca gacugauguu ga                                                22


<210> 6
<211> 21
<212> RNA
<213> Homo sapiens

<220>
<221> source
<222> 1..21
<223> /mol_type="RNA"
      /organism="Homo sapiens"

<220>
<221> ncRNA

<222> 1..21
<223> /ncRNA_class="siRNA"
      /note="SEQ ID NO: 6 = hsa-miR-23a"

<400> 6
aucacauugc cagggauuuc c                                                21


<210> 7
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 7 =hsa-miR-30b-3p"

<400> 7
cugggaggug gauguuuacu uc                                               22


<210> 8
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 8 = hsa-miR-129*"

<400> 8
aagcccuuac cccaaaaagc au                                               22


<210> 9
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"

/note="SEQ ID NO: 9 = hsa-miR-132"

<400> 9
uaacagucua cagccauggu cg                                              22


<210> 10
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 10 =hsa-miR-135a"

<400> 10
uauggcuuuu uauuccuaug uga                                             23


<210> 11
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 11 = hsa-miR-135b"

<400> 11
uauggcuuuu cauuccuaug uga                                             23


<210> 12
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 12 = hsa-miR-138"

<400> 12
agcugguguu gugaaucagg ccg                                              23


<210> 13
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 13 = hsa-miR-181a"

<400> 13
aacauucaac gcugucggug agu                                              23


<210> 14
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 14 =hsa-miR-181b "

<400> 14
aacauucauu gcugucggug ggu                                              23


<210> 15
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 15 = hsa-miR-181c"

<400> 15
aacauucaac cugucgguga gu                                               22

<210> 16
<211> 23
<212> RNA
<213> Homo sapiens

<220>
<221> source
<222> 1..23
<223> /mol_type="RNA"
      /organism="Homo sapiens"

<220>
<221> ncRNA
<222> 1..23
<223> /ncRNA_class="siRNA"
      /note="SEQ ID NO: 16 = hsa-miR-181d"

<400> 16
aacauucauu guugucggug ggu                                              23


<210> 17
<211> 21
<212> RNA
<213> Homo sapiens

<220>
<221> source
<222> 1..21
<223> /mol_type="RNA"
      /organism="Homo sapiens"

<220>
<221> ncRNA
<222> 1..21
<223> /ncRNA_class="siRNA"
      /note="SEQ ID NO: 17 = rno/mmu-miR-341"

<400> 17
ucggucgauc ggucggucgg u                                                21


<210> 18
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 18 = hsa-miR-374"

<400> 18
auauaauaca accugcuaag ug                                               22

<210> 19
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 19 = hsa-miR-375"

<400> 19
uuuguucguu cggcucgcgu ga                                              22


<210> 20
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 20 = hsa-miR-539"

<400> 20
ggagaaauua uccuuggugu gu                                              22


<210> 21
<211> 22
<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 21 = hsa-miR-758"

<400> 21
uuugugaccu gguccacuaa cc                                              22


<210> 22
<211> 22

<212> RNA
<213> Homo sapiens

<220>
<221> source
<222> 1..22
<223> /mol_type="RNA"
      /organism="Homo sapiens"

<220>
<221> ncRNA
<222> 1..22
<223> /ncRNA_class="siRNA"
      /note="SEQ ID NO: 22 = hsa-miR-592"

<400> 22
uugugucaau augcgaugau gu                                              22


<210> 23
<211> 21
<212> RNA
<213> Artificial Sequence

<220>
<221> source
<222> 1..21
<223> /mol_type="RNA"
      /note="SEQ ID NO: 23 = anti-hsa-miR-21*"
      /organism="Artificial Sequence"

<400> 23
acagcccauc gacugguguu g                                               21


<210> 24
<211> 22
<212> RNA
<213> Artificial Sequence

<220>
<221> source
<222> 1..22
<223> /mol_type="RNA"
      /note="SEQ ID NO: 24  = anti-hsa-let-7a"
      /organism="Artificial Sequence"

<400> 24
aacuauacaa ccuacuaccu ca                                              22


<210> 25
<211> 22
<212> RNA
<213> Artificial Sequence

<220>
<221> source
<222> 1..22
<223> /mol_type="RNA"
      /note="SEQ ID NO:25  = anti-hsa-miR-9*"
      /organism="Artificial Sequence"

<400> 25
acuuucgguu aucuagcuuu au                                              22

<210> 26
<211> 23
<212> RNA
<213> Artificial Sequence

<220>
<221> source
<222> 1..23
<223> /mol_type="RNA"
      /note="SEQ ID NO: 26 = anti-hsa-miR-17-5p"
      /organism="Artificial Sequence"

<400> 26
cuaccugcac uguaagcacu uug                                              23


<210> 27
<211> 22
<212> RNA
<213> Artificial Sequence

<220>
<221> source
<222> 1..22
<223> /mol_type="RNA"
      /note="SEQ ID NO: 27 = anti-hsa-miR-21"
      /organism="Artificial Sequence"

<400> 27
ucaacaucag ucugauaagc ua                                               22


<210> 28
<211> 21
<212> RNA
<213> Artificial Sequence

<220>
<221> source
<222> 1..21
<223> /mol_type="RNA"
      /note="SEQ ID NO: 28 = anti-hsa-miR-23a"
      /organism="Artificial Sequence"

<400> 28
ggaaaucccu ggcaauguga u                                                21


<210> 29
<211> 22
<212> RNA
<213> Artificial Sequence

<220>
<221> source
<222> 1..22
<223> /mol_type="RNA"
      /note="SEQ ID NO:29 = anti-hsa-miR-30b-3p"
      /organism="Artificial Sequence"

<400> 29
gaaguaaaca uccaccuccc ag                                               22

<210> 30
<211> 22
<212> RNA
<213> Artificial Sequence

<220>
<221> source
<222> 1..22
<223> /mol_type="RNA"
      /note="SEQ ID NO:30  = anti-hsa-miR-129*"
      /organism="Artificial Sequence"

<400> 30
augcuuuuug ggguaagggc uu                                              22


<210> 31
<211> 22
<212> RNA
<213> Artificial Sequence

<220>
<221> source
<222> 1..22
<223> /mol_type="RNA"
      /note="SEQ ID NO:31  = anti-hsa-miR-132"
      /organism="Artificial Sequence"

<400> 31
cgaccauggc uguagacugu ua                                              22


<210> 32
<211> 23
<212> RNA
<213> Artificial Sequence

<220>
<221> source
<222> 1..23
<223> /mol_type="RNA"
      /note="SEQ ID NO: 32 = anti-hsa-miR-135a"
      /organism="Artificial Sequence"

<400> 32
ucacauagga auaaaaagcc aua                                             23


<210> 33
<211> 23
<212> RNA
<213> Artificial Sequence

<220>
<221> source
<222> 1..23
<223> /mol_type="RNA"
      /note="SEQ ID NO:33  = anti-hsa-miR-135b"
      /organism="Artificial Sequence"

<400> 33
ucacauagga augaaaagcc aua                                             23

<210> 34
<211> 23
<212> RNA
<213> Artificial Sequence

<220>
<221> source
<222> 1..23
<223> /mol_type="RNA"
      /note="SEQ ID NO:34  = anti-hsa-miR-138"
      /organism="Artificial Sequence"

<400> 34
cggccugauu cacaacacca gcu                                              23


<210> 35
<211> 23
<212> RNA
<213> Artificial Sequence

<220>
<221> source
<222> 1..23
<223> /mol_type="RNA"
      /note="SEQ ID NO:35  = anti-hsa-miR-181a"
      /organism="Artificial Sequence"

<400> 35
acucaccgac agcguugaau guu                                              23


<210> 36
<211> 23
<212> RNA
<213> Artificial Sequence

<220>
<221> source
<222> 1..23
<223> /mol_type="RNA"
      /note="SEQ ID NO:36  = anti-hsa-miR-181b"
      /organism="Artificial Sequence"

<400> 36
acccaccgac agcaaugaau guu                                              23


<210> 37
<211> 22
<212> RNA
<213> Artificial Sequence

<220>
<221> source
<222> 1..22
<223> /mol_type="RNA"
      /note="SEQ ID NO:37  = anti-hsa-miR-181c"
      /organism="Artificial Sequence"

<400> 37
acucaccgac agguugaaug uu                                               22


<210> 38

<211> 22
<212> RNA
<213> Artificial Sequence

<220>
<221> source
<222> 1..22
<223> /mol_type="RNA"
      /note="SEQ ID NO:38  = anti-hsa-miR-181d"
      /organism="Artificial Sequence"

<400> 38
acccaccgac aacaaugaug uu                                              22


<210> 39
<211> 21
<212> RNA
<213> Artificial Sequence

<220>
<221> source
<222> 1..21
<223> /mol_type="RNA"
      /note="SEQ ID NO:39  = anti-rno/mmu-miR-341"
      /organism="Artificial Sequence"

<400> 39
accgaccgac cgaucgaccg a                                               21


<210> 40
<211> 22
<212> RNA
<213> Artificial Sequence

<220>
<221> source
<222> 1..22
<223> /mol_type="RNA"
      /note="SEQ ID NO: 40 = anti-hsa-miR-374"
      /organism="Artificial Sequence"

<400> 40
cacuuagcag guuguauuau au                                              22


<210> 41
<211> 22
<212> RNA
<213> Artificial Sequence

<220>
<221> source
<222> 1..22
<223> /mol_type="RNA"
      /note="SEQ ID NO: 41 = anti-hsa-miR-375"
      /organism="Artificial Sequence"

<400> 41
ucacgcgagc cgaacgaaca aa                                              22


<210> 42
<211> 22

<212> RNA
<213> Artificial Sequence

<220>
<221> source
<222> 1..22
<223> /mol_type="RNA"
      /note="SEQ ID NO:42  = anti-hsa-miR-539"
      /organism="Artificial Sequence"

<400> 42
acacaccaag gauaauuucu cc                                              22


<210> 43
<211> 22
<212> RNA
<213> Artificial Sequence

<220>
<221> source
<222> 1..22
<223> /mol_type="RNA"
      /note="SEQ ID NO: 43 = anti-hsa-miR-758"
      /organism="Artificial Sequence"

<400> 43
gguuagugga ccaggucaca aa                                              22


<210> 44
<211> 22
<212> RNA
<213> Artificial Sequence

<220>
<221> source
<222> 1..22
<223> /mol_type="RNA"
      /note="SEQ ID NO: 44 = anti-hsa-miR-592"
      /organism="Artificial Sequence"

<400> 44
acaucaucgc auauugacac aa                                              22


<210> 45
<211> 20
<212> RNA
<213> Artificial Sequence

<220>
<221> source
<222> 1..20
<223> /mol_type="RNA"
      /note="SEQ ID NO:45  = primer for rno-U6"
      /organism="Artificial Sequence"

<400> 45
cgcaaauucg ugaagcguuc                                                 20


<210> 46
<211> 23
<212> RNA

<213> Artificial Sequence

<220>
<221> source
<222> 1..23
<223> /mol_type="RNA"
      /note="SEQ ID NO:46  = primer for RNU43"
      /organism="Artificial Sequence"

<400> 46
cuuauugacg ggcggacaga aac                                              23


<210> 47
<211> 24
<212> RNA
<213> Artificial Sequence

<220>
<221> source
<222> 1..24
<223> /mol_type="RNA"
      /note="SEQ ID NO: 47 = primer for U1"
      /organism="Artificial Sequence"

<400> 47
cgacugcaua auuuguggua gugg                                             24